# EUROPEAN PATENT APPLICATION

(11) **EP 1 834 641 A1**
(43) Date of publication of application: **19.09.2007**
(21) Application number: 06290427.1
(22) Date of filing: 16.03.2006
(51) Int. Cl.: A61K 31/426, A61P 3/04, A61P 3/06, A61P 3/10

(54) **Use of CRF1 receptor antagonists for preparing a drug for treating metabolic syndrome and/or obesity and/or dyslipoproteinemia**

(71) Applicant: Sanofi-Aventis, 75013 Paris (FR)
(72) Inventor: Richard, Denis, GS1 1R4 Québec (CA)
(74) Representative: Kugel, Dominique

(57) **Abstract**

An object of the present invention is the use of a compound of formula (I): 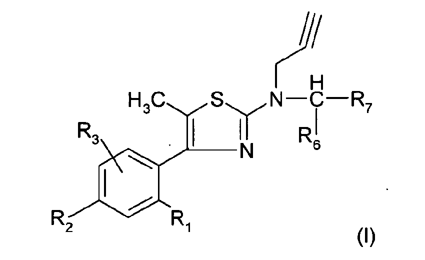 or one of its pharmaceutically acceptable salts,
for the manufacture of a medicament for the prevention and/or the treatment of metabolic syndrome and/or obesity and/or dyslipoproteinemia.

## Description

The present invention concerns the use of aminothiazole derivatives for preparing drugs, intended for preventively or curatively treating metabolic syndrome and/or obesity and/or dyslipoproteinemia.

The European patent n° EP 1 200 419 describes aminothiazole derivatives of formula (I), as CRF₁ antagonists: 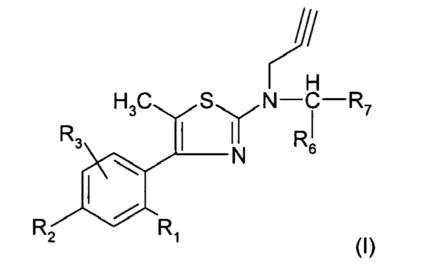 wherein
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂; or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cydoalkyl(C₁-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or -CN, with a group - COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof.

EP 1 200 419 also describes the use of the said compounds of formula (I) for the preparation of a medicament intended for preventively or curatively treating stress-related conditions and more particularly Cushing's disease, neuropsychiatric disorders such as depression, anxiety, panic, obessive compulsive disorders, mood disorders, post-traumatic stress, behavioural disorders, aggressiveness, anorexia, bulimia, hyperglycaemia, premature labour, at-risk pregnancy, retarded growth, sleeping disorders, epilepsy, and all types of depression; Alzheimer's disease, Parkinson's disease, Huntington's chorea; amyotrophic lateral sclerosis; vascular, cardiac and cerebral disorders; sexual activity disorders and fertility disorders; immunodepression, immunosuppression, inflammatory processes, multiple infections, rheumatoid arthritis, osteoarthritis, uveitis, psoriasis and diabetes; cancers; gastrointestinal functional disorders and inflammations arising therefrom, for instance irritable and inflammatory bowel, diarrhoea; pain-perception disorders, fibromyalgias which may or may not be associated with sleeping disorders, fatigue or migraine; symptoms associated with alcohol dependency and withdrawal from drugs.

It has now been found that the said compounds of formula (I) are therapeutically effective as active principle intended for preventively or curatively treating metabolic syndrome and/or obesity and/or dyslipoproteinemia.

Thus, an object of the present invention is the use of the compounds of formula (I) for the manufacture of a medicament intended for preventively or curatively treating metabolic syndrome and/or obesity and/or dyslipoproteinemia.

Metabolic syndrome, also known as syndrome X, encompasses a complex of disturbances of carbohydrate and fat metabolism characterized by obesity, dyslipoproteinemia (low HDL and high LDL, VLDL and triglycerides), hyperinsulinemia, insulin resistance, glucose intolerance and hypertension (Atherosclerosis X, F.P. Woodford, J. Davignon, A. Sniderman (Eds.), Elsevier Science BV, Amsterdam (1995): 520-524). Syndrome X seems to be responsible of energy imbalance. The dominant underlying risk factors for this syndrome appear to be abdominal obesity and insulin resistance. Insulin resistance is a generalized metabolic disorder, in which the body can't use insulin efficiently. For these reasons the metabolic syndrome is also called the insulin resistance syndrome.

As of today, there are no well-accepted criteria for diagnosing the metabolic syndrome.

However, the criteria proposed by the National Cholesterol Education Program (NCEP) Adult Treatment Panel III (ATP 111), are currently recommended and widely used: the patients suffering from metabolic syndrome present at least 3 of the following criteria: obesity, dyslipidemia, hypertension and hyperglycemia.

The American Heart Association and the National Heart, Lung, and Blood Institute recommend that the metabolic syndrome be identified as the presence of three or more of the following parameters:
- Elevated waist circumference:
   Men: Equal to or greater than 40 inches (102 cm)
   Women: Equal to or greater than 35 inches (88 cm)
- Elevated triglycerides:
   Equal to or greater than 150 mg/dL
- Reduced HDL ("good") cholesterol:
   Men: Less than 40 mg/dL
   Women: Less than 50 mg/dL
- Elevated blood pressure:
   equal to or greater than 130/85 mm Hg
- Elevated fasting glucose:
   Equal to or greater than 100 mg/dL

An object of the present invention is the use of a compound of formula (1): 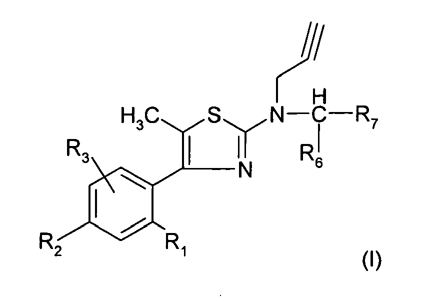 wherein
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁,-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂; or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cycloalkyl(Cₗ-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or -CN, with a group - COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof,
for the manufacture of a medicament intended for preventively or curatively treating metabolic syndrome.

The compounds of formula (I) can be provided in the form of a free base or in the form of addition salts with acids, which also form part of the invention.

The compounds of formula (I) can comprise one or more asymmetric carbon atoms. They can therefore exist in the form of enantiomers or diastereoisomers. These enantiomers and diastereoisomers, as well as their mixtures, including racemic mixtures, form part of the invention.

The compounds of formula (I) can also exist in the form of an hydrate or of a solvate, i.e. in the form of associations or combinations with one or more water or solvent molecules.

According to the present invention, the terms below have the following meanings:
- a halogen atom corresponds to a fluorine, chlorine, bromine or iodine atom;
- (Cₜ-C_{z}) represents a chain or a ring, which may contain from t to z carbon atoms and t and z may represent an integer chosen from 1 to 10. For examples, (C₁-C₃) represents a chain, which may contain from 1 to 3 carbon atoms and (C₃-C₆) represents a ring, which may contain from 3 to 6 carbon atoms ;
- an alkyl group corresponds to a saturated, linear or branched aliphatic group. The following examples may be cited: methyl, ethyl, propyl, isopropyl, butyl, isobutyl, tertbutyl, pentyl;
- a cycloalkyl group corresponds to a cyclic alkyl group. The following examples may be cited: cyclopropyl, methylcyclopropyl, cyclobutyl, cyclopentyl, cyclohexyl;
- an alkoxy group corresponds to an -O-alkyl group, wherein the alkyl group is as defined above.
- a hydroxyalkyl group corresponds to an alkyl group, wherein one or more hydrogen atoms have been substituted by hydroxyl group;
- an aryl group corresponds to an aromatic, cyclic group comprising between 5 and 6 carbon atoms, such as phenyl.
- an aralkyl group corresponds to an aryl group susbstituted on an alkylène chain. For example a benzyl group;

Another embodiment of the present invention concerns the use of a compound of formula (I), wherein
- R₁ and R₂, which may be identical or different, each independently represent a halogen atom; a (C₁-C₅)alkyl; a (C₁-C₅)alkoxy;
- R₃ represents hydrogen or is as defined above for R₁ and R₂;
- R₆ represents a (C₁-C₆)alkyl; a (C₁-C₆)alkoxy(C₁-C₃)alkyl; a (C₃-C₅)cycloalkyl; a (C₃-C₆)cydoalkyl(C₁-C₆)alkyl;
- R₇ represents a phenyl which is unsubstituted or mono- or disubstituted in position 3 or 4 with a halogen, a (C₁-C₅)alkyl group, a group -CH₂OR₈ in which R₈ represents a (C₁-C₃)alkyl or with an -O-CH₂-O- group in position 3, 4; or alternatively R₇ represents a (C₃-C₅)cycloalkyl group;
as well as the addition salts thereof, the hydrates thereof and/or the solvates thereof, for the manufacture of a medicament intended for preventively or curatively treating metabolic syndrome.

Another embodiment of the present invention concerns the use of a compound of formula (I), wherein R₃ is in position 5 of the phenyl, as well as the addition salts thereof, the hydrates thereof and/or the solvates thereof, for the manufacture of a medicament intended for preventively or curatively treating metabolic syndrome.

Another embodiment of the present invention concerns the use of a compound of formula (I), the said compound of formula (I) being chosen from:
- [4-(2-chloro-4-methoxy-5-methylphenyl)-5-methylthiazol-2-yl][(1 R)-(1 -(3-fluoro-4-methylphenyl)-2-methoxyethyl)]prop-2-ynylamine hydrochloride,
- [4-(2-chloro-4-methoxy-5-methylphenyl)-5-methylthiazol-2-yl][(1S)-(1-phenylbutyl)]prop-2-ynylamine hydrochloride,
- [4-(2-chloro-4-methoxy-5-methylphenyl)-5-methylthiazol-2-yl][(1 S)-(2-cyclopropyl-1-phenylethyl)]prop-2-ynylamine hydrochloride,
- [4-(2-chloro-4-methoxyphenyl)-5-methylthiazol-2-yl][(1 S)-(2-cyctopropy)-1-phenylethyl)]prop-2-ynylamine hydrochloride,
- [4-(2-chloro-4-methoxy-5-methylphenyl)-5-methylthiazol-2-yl][(1S)-(2-cyclopropyl-1-(4-fluorophenyl)ethyl)]prop-2-ynylamine hydrochloride,
- [4-(2-chloro-4-methoxy-5-methylphenyl)-5-methylthiazol-2-yl][(1 S)-(1-phenylpentyl)]prop-2-ynylamine hydrochloride,
- [4-(2-chloro-4-methoxy-5-methylphenyl)-5-methylthiazol-2-yl][(1R)-(2-methoxy-1-(4-methoxymethylphenyl)ethyl)]prop-2-ynylamine hydrochloride,
- [4-(2-chloro-4-methoxy-5-methylphenyl)-5-methylthiazol-2-ylj[(1S)-(1-(4-methoxymethylphenyl)pentyl)]prop-2-ynylamine hydrochloride,
- [4-(2-chloro-4-methoxy-5-methylphenyl)-5-methylthiazol-2-yl][(I S)-(1 -(4-fluorophenyl)pentyl)]prop-2-ynylamine hydrochloride,
- [4-(2-chloro-4-methoxy-5-methylphenyl)-5-methylthiazol-2-yl][(1S)-(cyclopropylphenylmethyl)]prop-2-ynylamine hydrochloride,
- [4-(2-chloro-4-methoxy-5-methylphenyl)-5-methylthiazol-2-yl][(1S)-(1-(3-fluoro-4-methylphenyl)pentyl)]prop-2-ynylamine hydrochloride,
- [4-(2-chloro-4-methoxy-5-methylphenyl)-5-methylthiazol-2-yl(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1 -(4-fluorophenyl)butyl)]prop-2-ynylamine hydrochloride,
- [4-(2-chloro-4-methoxy-5-methylphenyl)-5-methylthiazol-2-yl][(1S)-(1-(3-fluoro-4-methoxymethylphenyl)butyl)]prop-2-ynylamine hydrochloride,
- [4-(2-chloro-4-methoxy-5-methylphenyl)-5-methylthiazol-2-yl][(1 S)-(2-cyclopropyl-1-(4-chlorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-methylphenyl)ethyl)]prop-2-ynylamine hydrochloride,
- [4-(2-chloro-4-methoxy-5-methyl phenyl)-5-methylthiazol-2-yl][(1S)-(2-cyctobuty)-1 -(4-fluorophenyl)ethyl)]prop-2-ynylamine hydrochloride,
- [4-(2-chloro-4-methoxy-5-methylphenyl)-5-methylthiazol-2-yl][(I S)-(2-cyc)opropy)-1 - (4-bromophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(3,4-methylenedioxyphenyl)ethyl)]prop-2-ynylamine hydrochloride,
- [4-(2-ch)oro-4-methoxyphenyl)-5-methylthiazol-2-y)][(1S)-(2-cyclopropyl-1 -(3-fluoro-4-methylphenyl)ethyl)]prop-2-ynylamine hydrochloride,
- [4-(2,4-dimethoxy-5-methylphenyl)-5-methylthiazol-2-yl][(1S)-(2-cyclopropyl-1-(3-fluoro-4-methylphenyl)ethyl)]prop-2-ynylamine hydrochloride,
- (4-(4-methoxy-2,5-dimethylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1-(3,4-methylenedioxyphenyl)butyl)]prop-2-ynylamine hydrochloride,
as well as the addition salts thereof, the hydrates thereof and/or the solvates thereof, for the manufacture of a medicament intended for preventively or curatively treating metabolic syndrome.

Another embodiment of the present invention concerns the use of a compound of formula (I), wherein the said compound is [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride, for the manufacture of a medicament intended for preventively or curatively treating metabolic syndrome.

Another object of the invention is the use of a compound of formula (I): 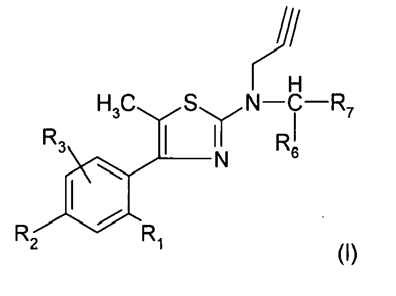 wherein
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂; or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or -CN, with a group - COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof, for the manufacture of a medicament intended for preventively or curatively treating obesity.

Another embodiment of the invention concerns the use of a compound of formula (I) wherein
- R₁ and R₂, which may be identical or different, each independently represent a halogen atom; a (C₁-C₅)alkyl; a (C₁-C₅)alkoxy;
- R₃ represents hydrogen or is as defined above for R₁ and R₂;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl;
- R₇ represents a phenyl which is unsubstituted or mono- or disubstituted in position 3 or 4 with a halogen, a (C₁-C₅)alkyl group, a group -CH₂OR₈ in which R₈ represents a (C₁-C₃)alkyl or with an -O-CH₂-O- group in position 3, 4; or alternatively R₇ represents a (C₃-C₅)cycloalkyl group;
as well as the addition salts thereof, the hydrates thereof and/or the solvates thereof, for the manufacture of a medicament intended for preventively or curatively treating obesity.

Another embodiment of the present invention concerns the use of a compound of formula (I), wherein R₃ is in position 5 of the phenyl, as well as the addition salts thereof, the hydrates thereof and/or the solvates thereof, for the manufacture of a medicament intended for preventively or curatively treating obesity.

Another embodiment of the present invention concerns the use of a compound of formula (I), the said compound of formula (I) being chosen from:
- [4-(2-chloro-4-methoxy-5-methylphenyl)-5-methylthiazol-2-yl][(1R)-(1-(3-fluoro-4-methylphenyl)-2-methoxyethyl)]prop-2-ynylamine hydrochloride,
- [4-(2-chloro-4-methoxy-5-methylphenyl)-5-methylthiazol-2-yl][(1S)-(1-phenylbutyl)]prop-2-ynylamine hydrochloride,
- [4-(2-chloro-4-methoxy-5-methylphenyl)-5-methylthiazol-2-yl][(1S)-(2-cyclopropyl-1-phenylethyl)]prop-2-ynylamine hydrochloride,
- [4-(2-chloro-4-methoxyphenyl)-5-methylthiazol-2-yl][(1S)-(2-cyclopropyl-1-phenylethyl)]prop-2-ynylamine hydrochloride,
- [4-(2-chloro-4-methoxy-5-methylphenyl)-5-methylthiazol-2-yl][(1S)-(2-cyclopropyl-1-(4-fluorophenyl)ethyl)]prop-2-ynylamine hydrochloride,
- [4-(2-ch)oro-4-methoxy-5-methytphenyl)-5-methylthiazol-2-yt][(1 S)-(1-phenylpentyl)]prop-2-ynylamine hydrochloride,
- [4-(2-chloro-4-methoxy-5-methylphenyl)-5-methylthiazol-2-yl][(1 R)-(2-methoxy-1-(4-methoxymethylphenyl)ethyl)]prop-2-ynylamine hydrochloride,
- [4-(2-chloro-4-methoxy-S-methylphenyl)-5-methylthiazol-2-yl][(1 S)-(1-(4-methoxymethylphenyl)pentyl)]prop-2-ynylamine hydrochloride,
- [4-(2-chloro-4-methoxy-5-methylphenyl)-5-methylthiazol-2-yl][(1S)-(1 -(4-fluorophenyl)pentyl)]prop-2-ynylamine hydrochloride,
- [4-(2-chloro-4-methoxy-5-methylphenyl)-5-methylthiazol-2-yl][(1 S)-(cyclopropylphenylmethyl)]prop-2-ynylamine hydrochloride,
- [4-(2-chloro-4-methoxy-5-methylphenyl)-5-methylthiazol-2-yl][(1S)-(1 -(3-fluoro-4-methylphenyl) pentyl)] prop-2-ynylamine hydrochloride,
- [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1-(4-fluorophenyl)butyl)]prop-2-ynylamine hydrochloride,
- [4-(2-chloro-4-methoxy-5-methylphenyl)-5-methylthiazol-2-yl][(1S)-(1-(3-fluoro-4-methoxymethylphenyl)butyl)]prop-2-ynylamine hydrochloride,
- [4-(2-chloro-4-methoxy-5-methylphenyl)-5-methylthiazol-2-yl][(1S)-(2-cyclopropyl-1-(4-chlorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-methylphenyl)ethyl)]prop-2-ynylamine hydrochloride,
- [4-(2-chloro-4-methoxy-5-methylphenyl)-5-methylthiazol-2-yl][(1S)-(2-cyclobutyl-1-(4-fluorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-bromophenyl)ethyl)]prop-2-ynylamine hydrochloride,
- [4-(2 -chloro-4-methoxy-S-methylphenyl)-S-methylthiazol-2-yl] [(1S)-(2-cydopropyl-1 - (3,4-methylenedioxyphenyl)ethyl)]prop-2-ynylamine hydrochloride,
- [4-(2-chloro-4-methoxyphenyl)-5-methylthiazol-2-yl][(1S)-(2-cyclopropyl-1-(3-fluoro-4-methylphenyl)ethyl)]prop-2-ynylamine hydrochloride,
- [4-(2,4-dimethoxy-S-methylphenyl)-S-methylthiazol-2-yl][(1S)-(2-cydopropyl-1 -(3-fluoro-4-methylphenyl)ethyl)]prop-2-ynylamine hydrochloride,
- [4-(4-methoxy-2,5-dimethylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-S-methylphenyl)-S-methylthiazol-2-yl][(1S)-(1-(3,4-methylenedioxyphenyl)butyl)]prop-2-ynylamine hydrochloride,
as well as the addition salts thereof, the hydrates thereof and/or the solvates thereof, for the manufacture of a medicament intended for preventively or curatively treating obesity.

Another embodiment of the present invention concerns the use of a compound of formula (I), wherein the said compound is [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride, for the manufacture of a medicament intended for preventively or curatively treating obesity.

Another object of the invention is the use of a compound of formula (I): 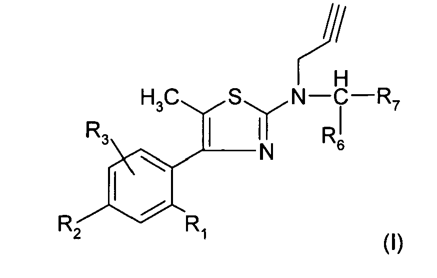 wherein
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂; or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or-CN, with a group - COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof, for the manufacture of a medicament intended for preventively or curatively treating dyslipoproteinemia.

Another embodiment of the invention concerns the use of a compound of formula (I) wherein
- R₁ and R₂, which may be identical or different, each independently represent a halogen atom; a (C₁-C₅)alkyl; a (C₁-C₅)alkoxy;
- R₃ represents hydrogen or is as defined above for R₁ and R₂;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl;
- R₇ represents a phenyl which is unsubstituted or mono- or disubstituted in position 3 or 4 with a halogen, a (C₁-C₅)alkyl group, a group -CH₂OR₈ in which R₈ represents a (C₁-C₃)alkyl or with an -O-CH₂-O- group in position 3, 4; or alternatively R₇ represents a (C₃-C₅)cycloalkyl group;
as well as the addition salts thereof, the hydrates thereof and/or the solvates thereof, for the manufacture of a medicament intended for preventively or curatively treating dyslipoproteinemia.

Another embodiment of the present invention concerns the use of a compound of formula (I), wherein R₃ is in position 5 of the phenyl, as well as the addition salts thereof, the hydrates thereof and/or the solvates thereof, for the manufacture of a medicament intended for preventively or curatively treating dyslipoproteinemia.

Another embodiment of the present invention concerns the use of a compound of formula (I), the said compound of formula (I) being chosen from:
- [4-(2-chloro-4-methoxy-5-methylphenyl)-5-methylthiazol-2-yl][(1 R)-(1 1-(3-fl uoro-4-methylphenyl)-2-methoxyethyl)]prop-2-ynylamine hydrochloride,
- [4-(2-chloro-4-methoxy-5-methylphenyl)-5-methylthiazol-2-yl][(1S)-(1 - phenylbutyl)]prop-2-ynylamine hydrochloride,
- [4-(2-chloro-4-methoxy-5-methylphenyl)-5-methylthiazol-2-yl][(1S)-(2-cyclopropyl-1-phenylethyl)]prop-2-ynylamine hydrochloride,
- [4-(2-chloro-4-methoxyphenyl)-5-methylthiazol-2-yl][(1S)-(2-cyclopropyl-1-phenylethyl)]prop-2-ynylamine hydrochloride,
- [4-(2-chloro-4-methoxy-5-methylphenyl)-5-methylthiazol-2-yl][(1S)-(2-cyclopropyl-1-(4-fluorophenyl)ethyl)]prop-2-ynylamine hydrochloride,
- [4-(2-chloro-4-methoxy-5-methylphenyl)-5-methylthiazol-2-yl][(1S)-(1-phenylpentyl)]prop-2-ynylamine hydrochloride,
- [4-(2-chloro-4-methoxy-5-methylphenyl)-5-methylthiazol-2-yl][(1R)-(2-methoxy-1-(4-methoxymethylphenyl)ethyl)]prop-2-ynylamine hydrochloride,
- [4-(2-chloro-4-methoxy-5-methylphenyl)-5-methylthiazol-2-yl][(1S)-(1 -(4-methoxymethylphenyl)pentyl)]prop-2-ynylamine hydrochloride,
- [4-(2-chloro-4-methoxy-5-methylphenyl)-5-methylthiazol-2-yl][(1S)-(1-(4-fluorophenyl)pentyl)]prop-2-ynylamine hydrochloride,
- [4-(2-chloro-4-methoxy-5-methylphenyl)-5-methylthiazol-2-yl][(1 S)-(cyclopropylphenylmethyl)]prop-2-ynylamine hydrochloride,
- [4-(2-chloro-4-methoxy-5-methylphenyl)-5-methylthiazol-2-yl][(1S)-(1-(3-fluoro-4-methylphenyl)pentyl)]prop-2-ynylamine hydrochloride,
- [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1-(4-fluorophenyl)butyl)]prop-2-ynylamine hydrochloride,
- [4-(2-chloro-4-methoxy-5-methylphenyl)-5-methylthiazol-2-yl][(1S)-(1-(3-fluoro-4-methoxymethylphenyl)butyl)]prop-2-ynylamine hydrochloride,
- (4-(2-chloro-4-methoxy-5-methylphenyl)-5-methylthiazol-2-yl][(1S)-(2-cyclopropyl-1-(4-chlorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-methylphenyl)ethyl)]prop-2-ynylamine hydrochloride,
- [4-(2-chloro-4-methoxy-5-methylphenyl)-5-methylthiazol-2-yl][(1S)-(2-cyclobutyl-1-(4-fluorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-bromophenyl)ethyl)]prop-2-ynylamine hydrochloride,
- [4-(2-chloro-4-methoxy-5-methylphenyl)-5-methylthiazol-2-yl][(1S)-(2-cydopropyl-1 - (3,4-methylenedioxyphenyl)ethyl)]prop-2-ynylamine hydrochloride,
- [4-(2-chloro-4-methoxyphenyl)-5-methylthiazol-2-yl][(1S)-(2-cyclopropyl-1-(3-fluoro-4-methylphenyl)ethyl)]prop-2-ynylamine hydrochloride,
- [4-(2,4-dimethoxy-5-methylphenyl)-5-methylthiazol-2-yl][(1S)-(2-cyclopropyl-1-(3-fluoro-4-methylphenyl)ethyl)]prop-2-ynylamine hydrochloride,
- [4-(4-methoxy-2,5-dimethylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1-(3,4-methylenedioxyphenyl)butyl)]prop-2-ynylamine hydrochloride,
as well as the addition salts thereof, the hydrates thereof and/or the solvates thereof, for the manufacture of a medicament intended for preventively or curatively treating dyslipoproteinemia.

Another embodiment of the present invention concerns the use of a compound of formula (I), wherein the said compound is [4-(2-chloro-4-methoxy-5-methylphenyl)-5-methylthiazol-2-yl][(1 S)-(2-cyclopropyl-1-(3-fluoro-4-methylphenyl)ethyl)]prop-2-ynylamine hydrochloride, for the manufacture of a medicament intended for preventively or curatively treating dyslipoproteinemia.

The present invention concerns a method for preventively or curatively treating metabolic syndrome and/or obesity and/or dyslipoproteinemia, which comprises administering to a mammal a therapeutically effective amount of at least a compound of general formula (I).

The preparation and the characteristics of compounds of formula (I) according to the invention have been described in European patent No. EP 1 200 419.

The compounds of the invention underwent pharmacological studies (determination of physiological parameters) which demonstrate their properties, and their value as therapeutically active substances, namely for the treatment and prevention of metabolic syndrome and/or obesity and/or dyslipoproteinemia.

More particularly, compounds of general formula (I) have been tested, in order to examine the effects on some representative physiological parameters and on energy balance.

The properties of the compounds of general formula (I) have been investigated by means of a set of tests in animals conventionally employed in pharmacology. For instance, the genetically obese (*fa*/*fa*) Zucker rat is one of the most investigated models of obesity. The fa mutation prevents the expression of the long isoform of the leptin receptor (Phillips et al, Nat. Genet.1996; 13: 18-19), which mediates the anorectic and thermogenic actions of leptin. The (fa/fa) rat is characterized by a massive obesity that develops early after birth, resulting from an increased food intake and a blunted regulatory thermogenesis. In addition, it is hyperlipidemic, hyperinsulinemic, glucose intolerant, and insulin resistant. It also has very high levels of corticosterone due to an overstimulated hypothalamo-pituitary-adrenal (HPA) axis (Guillaume-Gentil et al, Endocrinology 1990; 126: 1873-1879) and overexpresses the corticotropin-releasing factor (CRF) when faced with stressful experimental conditions, e.g. food deprivation (Guillaume-Gentil et al, 1990; 126: 1873-1879; Timofeeva and Richard, J. Comp. Neurol. 1997; 441: 71-89).

The following TESTS 1 and 2 have been performed with one of the compound of the present invention, which is [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride (= compound A).

### Materials and methods

### 1. Rats used for the experiments

Lean (Fa/?) and obese (fa/fa) male Zucker rats, aged 8-9 weeks, were purchased from Charles River Laboratories (St-Constant, QC). All rats were cared for and handled according to the Canadian Guide for the Care and Use of Laboratory Animals, and the protocol was approved by University Laval animal care committee. Throughout the study, rats were given a purified, high-carbohydrate diet, which was composed of the following (in g/100 g): 31.2 cornstarch, 31.2 DL-dextrose, 6.4 soybean oil, 20.0 casein, 0.3 DL-methionine, 1.0 vitamin mix (Teklad no. 40060; Teklad, Madison, WI), 4.9 AIN-93 mineral mix (ICN Biochemicals, Montréal, QC), and 5.0 fiber (Alphacel; ICN Biochemicals). The energy content of the diet consisted of 64.9% carbohydrate, 14.5% fat, and 20.6% protein, and its density was 4.01 kcal/g. Rats were subjected to a 12h/12 h dark/light cycle (light on between 07h00 and 19h00 and dark on between 19h00 and 07h00) and kept under an ambient temperature of 23 ± 1 °C. Treated rats received a two daily oral administration of Compound A for 21 days; 10 mg/kg at 08h30 and 20 mg/kg at 16h00. On the last day of treatment, 30 mg/kg Compound A was administered 4 hours prior to sacrifice.

Rats were weighted and food intake was measured daily throughout the experiment. Rats were killed between 14h00 and 17h00 in either an *ad libitum* fed state or after a 6-hour food deprivation. Lean and obese rats were respectively anaesthetized with 2 and 4 ml of a mixture containing 20 mg/ml of ketamine and 2.5 mg/ml of xylazine. Blood was collected by intracardial puncture into syringes coated with 0.5 M ethylenediaminetetraacetic acid (EDTA; Sigma-Aldrich, St. Louis, MO) and rats were perfused intracardially for 2 min with icecold isotonic saline.

### 2. Préparation of the solution containing compound A to be administred

Compound A was suspended in 0.6% methyl cellulose containing 0.5% Tween 80 for the first 14 days and in 5% DMSO (Dimethylsulfoxyde), 5% Cremophor EL, 90% saline for the last 7 days of treatment.

### 3. TEST 1: Measurements of body gains in energy, fat and protein

Carcasses were autoclaved at 125 kPa for 15 min to soften hard tissues, a procedure reported not to affect energy yield (Lofti *et al.* 1976). Carcass energy content was determined by adiabatic bomb calorimetry, whereas carcass protein was determined using a FP-2000 Nitrogen Analyser (Leco corporation, St. Joseph, MI) with 250-300 mg of dehydrated carcasses. Nonprotein matter energy was obtained by subtracting protein energy from total carcass energy. Because carbohydrate represents a negligible part of total carcass energy (Webster 1983), nonprotein matter energy was assumed to be essentially from fat. Values of 23.5 and 39.2 kj/g were used for the calculation of the energy content of protein and fat, respectively (Webster 1983). Initial energy, fat, and protein contents of the carcasses were estimated from the live body weight of lean and obese rats with reference to a baseline group of rats killed at the beginning of the experimental period. Such estimates allow gains in energy, fat, and protein to be determined for the treatment period. The rats in the baseline groups (six per phenotype) were killed at the beginning of the energy balance trial, and the carcass of each animal was analyzed for energy, protein, and fat. The densities in energy (kilocalories of energy per gram of body weight), protein (grams of protein per gram of body weight), and fat (grams of fat per gram of body weight) were then computed and averaged. The average densities were multiplied by the initial body weight of each rat ascribed to experimental groups. Rats in the initial group were identical in every respect (e.g., age and gender) to those of the experimental groups. Food efficiency was expressed as the ratio of energy gain to digestible energy intake multiplied by 100.

### 4. TEST 2: Plasma determinations

Blood was harvested by cardiac puncture, centrifuged (1500 g, 15 min at 4 °C), and the separated plasma was stored at -20 °C until later biochemical measurements. Plasma glucose concentrations were determined using an automated glucose analyzer YSI 2300 Stat Plus (YSI Incorporated, Yellow Springs, OH). Plasma triglycerides were assayed using a commercially available enzymatic kit (Roche Diagnostics) that allowed correction for free glycerol. Plasma insulin and leptin levels were determined using commercially available radioimmunoassay kits (Linco Research, St. Charles, MO). Plasma corticosterone levels were determined using a commercially available radioimmunoassay kit (MP Biomedicals, Toronto, ON). Plasma non-esterified fatty acids (NEFA) were assayed using a commercially available enzymatic kit (Wako Diagnostics, Richmond, VA).

### 5. Statistics

Results are presented as mean values ± one standard error of the mean (SE). Statistical differences in daily food intake and cumulative weight gain between control and compound A-treated rats were determined within each genotype by repeated-measures analysis of variance (RM-ANOVA) using a mixed model analysis. Cumulative weight gain data were log-transformed and multivariate normality was verified with Mardia's test. For the results of the carcasses analysis and HOMA-IR, statistical differences within each genotype were determined by Student's *t*-test. For all the other variables, statistical differences within each genotype were determined by two-way analysis of variance (ANOVA). In order to meet the assumption of normality, data for corticosterone, insulin and triglycerides were log transformed. All two-way ANOVAs were followed by Tukey's multiple comparison tests. Results were considered significant with P-values < 0.05. RM-ANOVAs were performed using SAS v9.1.3 software package (SAS Institute, Cary, NC), whereas all other statistical analyses used SigmaStat v2.0 software (SPSS, Chicago, IL).

The results of TESTS 1 and 2 (points 3 and 4), are illustrated by the following figures.

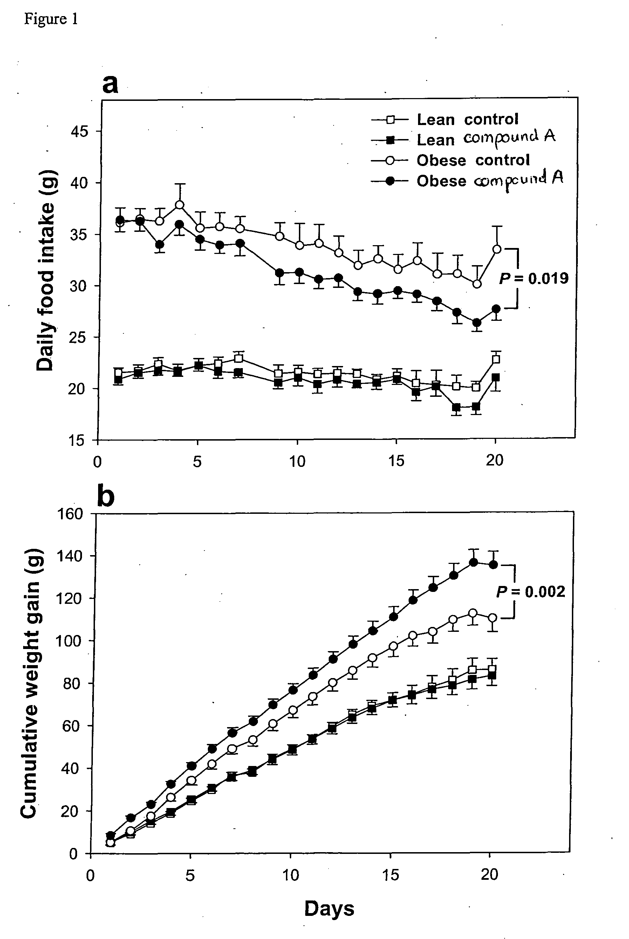

Figure 1. Daily food intake (a) and cumulative weight gain (b) in lean (Fa/?) and genetically obese (fa/fa) Zucker rats during 20 days of daily oral administration of 30 mg/kg compound A. * P < 0.05, n = 14 / group.

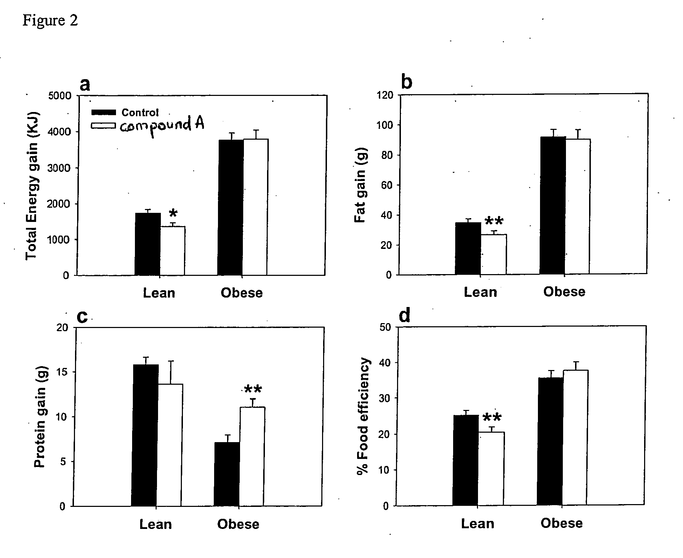

Figure 2. Total energy gain (a), protein gain (b), fat gain (c) and percentage food efficiency (d) in lean (Fa/?) and genetically obese *(falfa)* Zucker rats after 20 days of daily oral administration of 30 mg/kg compound A. * Significant effect of treatment as assessed by Student's *t*-test. P < 0.05, n = 7 / group.

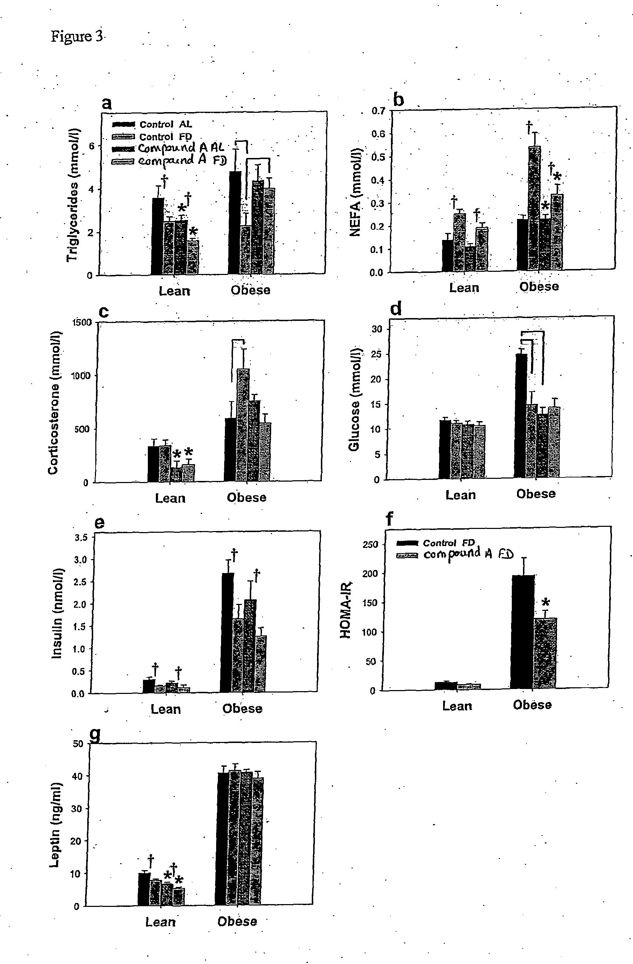

Figure 3. Plasma triglycerides (a), non-esterified fatty acids (NEFA) (b), corticosterone (c), glucose (d) and insulin (e) in *ad-libifum* fed (AL) and 6-hour food deprived (FD) lean (Fa/?) and genetically obese (fa/fa) Zucker rats after 20 days of daily oral administration of 30 mg/kg compound A. Homeostasis model assessment of insulin resistance (HOMA-IR) was calculated for FD rats using glucose and insulin measurements (f). * Significant main effect of treatment and t significant main effect of food deprivation as assessed by two-way ANOVA or *t*-test (HOMA-IR). When significant, only interaction results are shown (bars), as assessed by Tukey's multiple comparison tests. P < 0.05, n = 6-7 / group.

The results of TEST 1 are summed up in figures 1.a, 1.b, 2.a, 2.b, 2.c and 2.d. Treatment with compound A had significantly reduced daily food intake in obese but not in lean rats (Fig. 1a). Despite a reduced energy intake, compound A increased cumulative body weight gain in obese rats (Fig. 1 b) but this body weight gain was associated with an increased protein gain (Fig. 2c). Although compound A did not affect weight gain in lean rats, total energy and fat gains were reduced (Figs. 2a,b). The percentage food efficiency was also reduced in lean rats (Fig. 2d).

Consequently, compound A exhibits beneficial effects against some obesity parameters such as total energy, fat gains and food efficiency.

The results of TEST 2 are summed up in figures 3.a, 3.b, 3.c, 3.d, 3.e, 3.f and 3.g. Treatment with compound A reduced circulating triglyceride levels in lean rats (Fig. 3a). The fasting-induced reduction in plasma triglycerides was abolished by compound A in obese rats. Circulating non-esterified fatty acids (NEFA) were increased following food deprivation but this increase was blunted by compound A in obese rats (Fig. 3b). Compound A reduced plasma corticosterone levels in lean rats and prevented the fasting-induced increase in corticosterone levels in obese rats (Fig. 3c). Circulating glucose levels in compound A-treated obese rats were reduced to values similar to those of lean and untreated food-deprived obese rats (Fig. 3d). Plasma glucose and insulin levels of food-deprived rats were used to calculate the homeostasis model assessment of insulin resistance (HOMA-IR) (Matthews *et al.* 1985). Although compound A did not significantly modify plasma insulin levels (Fig. 3e), the HOMA-IR index was reduced in obese rats (Fig. 3f). Both the compound A and food deprivation reduced circulating leptin levels in lean but not in obese rats (Fig. 3g).

These results, namely:
- restoration of normal glycemia in obese rats and reduction of the HOMA-IR index of insulin resistance, pointing out an improved insulin sensitivity,
- prevention of fasting-induced increase in NEFA and of fasting-induced reduction of triglycerides in obese rats,
indicate that compound A has beneficial effects on plasma parameters related to the metabolic syndrome also called insulin resistance.

Compound A also decrease fasting-induced increase in corticosterone secretion in obese rats, which is in part responsible of energy deposition and inhibition of brown adipose tissue thermogenesis. Despite a reduced energy intake (daily food intake), compound A increases cumulative body weight gain in obese rats associated with an increased protein rather than fat gain.

Consequently, compounds of formula (I) according to the invention show beneficial effects in the treatment or prevention of metabolic syndrome and/or obesity and/or dyslipoproteinemia.
For the intended use disclosed in the invention, compounds of formula (I) may be comprised in pharmaceutical composition as active principle. These pharmaceutical compositions comprise an effective dose of one compound according to the invention, or an addition salt thereof with a pharmaceutically acceptable salt, or an hydrate or solvate of the latter, and at least one pharmaceutically acceptable excipient.
Said excipients are chosen according to the pharmaceutical form and the administration route desired, among usual excipients known of one of skill in the art.

In the corresponding pharmaceutical compositions for the oral, sublingual, sub-cutaneous, intramuscular, intra-venous, topical, local, intratracheal, intranasal, transdermal or rectal administration, the active principle of formula (I) above, its salt, solvate or hydrate, can be administered as a unitary dosage form, in blend with usual pharmaceutical excipients, to animals and human beings for the prevention or for the treatment of diseases mentioned above.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.
As an example, a unitary dosage form for a compound according to the invention, in the form of a tablet, can comprise the following ingredients:

| | |
|---|---|
| Compound according to the invention | 50,0 mg |
| Mannitol | 223,75 mg |
| Croscarmellose sodique | 6,0 mg |
| Maize starch | 15,0 mg |
| Hydroxypropyl methylcellulose | 2,25 mg |
| Magnesium stearate | 3,0 mg |

By the oral route, the dose of active principle to administer can reach 0.5 to 800 mg/kg, more preferably 0.5 to 200 mg/kg, per day, in one or several intakes.
In specific cases, higher or lower dosages may be appropriate; these dosages are comprised within the scope of the present invention. According to usual practice, the dosage suitable to each patient is determined by the physician according to the administration route, the weight and response of the patient.

## Claims

1. Use of a compound of formula (1): 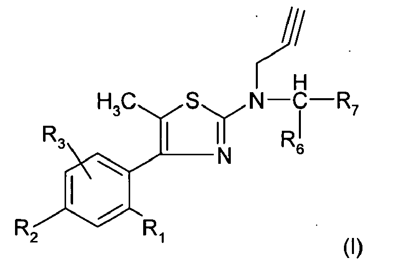 wherein
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂; or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or -CN, with a group - COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof, for the manufacture of a medicament intended for preventively or curatively treating metabolic syndrome.

2. Use according to claim 1 of a compound of formula (I), wherein
- R₁ and R₂, which may be identical or different, each independently represent a halogen atom; a (C₁-C₅)alkyl; a (C₁-C₅)alkoxy;
- R₃ represents hydrogen or is as defined above for R₁ and R₂;
- R₆ represents a (C₁-C₆)alkyl; a (C₁-C₆)aikoxy(C₁-C₃)alkyl; a (C₃-C₅)cycloalkyl; a (C₃-C₆)cydoalkyl(C₁-C₆)alkyl;
- R₇ represents a phenyl which is unsubstituted or mono- or disubstituted in position 3 or 4 with a halogen, a (C₁-C₅)alkyl group, a group -CH₂OR₈ in which R₈ represents a (C₁-C₃)alkyl or with an -O-CH₂-O- group in position 3, 4; or alternatively R₇ represents a (C₃-C₅)cycloalkyl group;
as well as the addition salts thereof, the hydrates thereof and/or the solvates thereof, for the manufacture of a medicament intended for preventively or curatively treating metabolic syndrome.

3. Use according to claims 1 or 2 of a compound of formula (I), wherein R₃ is in position 5 of the phenyl, as well as the addition salts thereof, the hydrates thereof and/or the solvates thereof, for the manufacture of a medicament intended for preventively or curatively treating metabolic syndrome.

4. Use according to claim 1, wherein the compound of formula (I) is chosen from:
- [4-(2-chloro-4-methoxy-5-methylphenyl)-5-methylthiazol-2-yl][(1 R)-(1-(3-fluoro-4-methylphenyl)-2-methoxyethyl)]prop-2-ynylamine hydrochloride,
- [4-(2-chloro-4-methoxy-5-methylphenyl)-5-methylthiazol-2-yl][(1S)-(1-phenylbutyl)]prop-2-ynylamine hydrochloride,
- [4-(2-chloro-4-methoxy-5-methylphenyl)-5-methylthiazol-2-yl] [(1 S)-(2-cyclopropy)-1 - phenylethyl)]prop-2-ynylamine hydrochloride,
- [4-(2-chloro-4-methoxyphenyl)-5-methylthiazol-2-yl][(1S)-(2-cyclopropyl-1-phenylethyl)]prop-2-ynylamine hydrochloride,
- [4-(2-chloro-4-methoxy-5-methylphenyl)-5-methylthiazol-2-yl][(1S)-(2-cyclopropyl-1-(4-fluorophenyl)ethyl)]prop-2-ynylamine hydrochloride,
- [4-(2-chloro-4-methoxy-5-methylphenyl)-5-methylthiazol-2-yl][(1S)-(1-phenylpentyl)]prop-2-ynylamine hydrochloride,
- [4-(2-chloro-4-methoxy-5-methylphenyl)-5-methylthiazol-2-yl][(1 R)-(2-methoxy-1-(4-methoxymethylphenyl)ethyl)]prop-2-ynylamine hydrochloride,
- [4-(2-chloro-4-methoxy-5-methylphenyl)-5-methylthiazol-2-yl][(1S)-(1-(4-methoxymethylphenyl)pentyl)]prop-2-ynylamine hydrochloride,
- [4-(2-chloro-4-methoxy-5-methylphenyl)-5-methylthiazol-2-yl][(1S)-(1 -(4-fluorophenyl)pentyl)]prop-2-ynylamine hydrochloride,
- [4-(2-chloro-4-methoxy-S-methylphenyl)-5-methylthiazol-2-yl][(1S)-(cyclopropylphenylmethyl)]prop-2-ynylamine hydrochloride,
- [4-(2-chloro-4-methoxy-5-methylphenyl)-5-methylthiazol-2-yl][(S)-(1-(3-fluoro-4-methylphenyl)pentyl)]prop-2-ynylamine hydrochloride,
- [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(I S)-(1 -(4-fluorophenyl)butyl)]prop-2-ynylamine hydrochloride,
- [4-(2-chloro-4-methoxy-5-methylphenyl)-5-methylthiazol-2-yl][(1 S)-(1 1-(3-fluoro-4-methoxymethylphenyl)butyl)]prop-2-ynylamine hydrochloride,
- [4-(2-chloro-4-methoxy-S-methylphenyl)-S-methylth iazol-2-yl][(1 S)-(2-cyclopropyl-1-(4-chlorophenyl)ethyl)]prop-2-ynylamine hydrochloride,
- [4-(2-chloro-4-methoxy-5-methylphenyl)-5-methylthiazol-2-yl] [(1S)-(2-cyclopropyl-1-(4-methylphenyl)ethyl)]prop-2-ynylamine hydrochloride,
- [4-(2-chloro-4-methoxy-5-methylphenyl)-5-methylthiazol-2-yl][(1S)-(2-cyclobutyl-1-(4-fluorophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(4-bromophenyl)ethyl)]prop-2-ynylamine hydrochloride,
- [4-(2-chloro-4-methoxy-5-methylphenyl)-5-methylthiazol-2-yl][(1S)-(2-cyclopropyl-1-(3,4-methylenedioxyphenyl)ethyl)]prop-2-ynylamine hydrochloride,
- [4-(2-chloro-4-methoxyphenyl)-5-methylthiazol-2-yl][(1S)-(2-cyclopropyl-1-(3-fluoro-4-methylphenyl)ethyl)]prop-2-ynylamine hydrochloride,
- [4-(2,4-dimethoxy-5-methylphenyl)-5-methylthiazol-2-yl][(1S)-(2-cyclopropyl-1-(3-fluoro-4-methylphenyl)ethyl)]prop-2-ynylamine hydrochloride,
- [4-(4-methoxy-2, S-dimethylphenyl)-S-methylthiazol-2-yl][(1S)-(2-cyclopropyl-1-(3-fluoro-4-methylphenyl)ethyl)]prop-2-ynylamine hydrochloride,
- [4-(2-chloro-4-methoxy-5-methylphenyl)-5-methylthiazol-2-yl][(1S)-(1-(3,4-methylenedioxyphenyl)butyl)]prop-2-ynylamine hydrochloride,
as well as the addition salts thereof, the hydrates thereof and/or the solvates thereof, for the manufacture of a medicament intended for preventively or curatively treating metabolic syndrome.

5. Use according to claim 1, wherein the compound of formula (I) is [4-(2-chloro-4-methoxy-5-methylphenyl)-5-methylthiazol-2-yl][(1 S)-(2-cyclopropyl-1-(3-fluoro-4-methylphenyl)ethyl)]prop-2-ynylamine hydrochloride, for the manufacture of a medicament intended for preventively or curatively treating metabolic syndrome.

6. Use of a compound of formula (I) as well as the addition salts thereof, the hydrates thereof and/or the solvates thereof, according to any one of claims 1, 2, 3, 4 of 5 for the manufacture of a medicament intended for preventively or curatively treating obesity.

7. Use of a compound of formula (I) as well as the addition salts thereof, the hydrates thereof and/or the solvates thereof, according to any one of claims 1, 2, 3, 4 of 5 for the manufacture of a medicament intended for preventively or curatively treating dyslipoproteinemia.
